# EUROPÄISCHE PATENTSCHRIFT

(11) **EP 0 941 106 B1**
(45) Veröffentlichungstag und Bekanntmachung des Hinweises auf die Patenterteilung: **27.02.2002**
(21) Anmeldenummer: 96946001.3
(22) Anmeldetag: 18.11.1996
(51) Int. Cl.: A61K 38/01, A61K 31/195, A23L 1/305, A61P 3/02, A61P 37/00

(54) **MITTEL ZUR METABOLISCHEN BEHANDLUNG DER MULTIPLEN SKLEROSE**
AGENT FOR THE METABOLIC TREATMENT OF MULTIPLE SCLEROSIS
AGENT POUR LE TRAITEMENT METABOLIQUE DE LA SCLEROSE EN PLAQUES

(43) Veröffentlichungstag der Anmeldung: 15.09.1999
(73) Patentinhaber: Kluge, Ralf, Dr., 06114 Halle (DE); Wasicki, Peter, Dr., 10409 Berlin (DE)
(72) Erfinder: KLUGE, Ralf, D-06114 Halle (DE); WASICKI, Peter, D-10409 Berlin (DE); WESTPHAL, Günter, D-10319 Berlin (DE)
(74) Vertreter: Walter, Wolf-Jürgen
(86) Internationale Anmeldenummer: DE9602228
(87) Internationale Veröffentlichungsnummer: WO9822123

(56) Entgegenhaltungen:
- EP-A- 0 390 716
- DE-A- 4 211 878
- DE-U- 29 516 585

## Beschreibung

Die Erfindung betrifft ein Mittel zur Behandlung von der multiplen Sklerose zugrundeliegenden Stoffwechselstörungen und der damit verbundenen energetischen Verarmung der Nervenzellen.

Nach dem gegenwärtigen Kenntnisstand handelt es sich bei der multiplen Sklerose (MS) um eine Autoimmunaggression gegenüber den Myelinscheiden. Eine begünstigende genetische Disposition ist sehr wahrscheinlich, es ist jedoch z.B. im Zusammenhang mit Autoimmunerkrankungen auf allgemein regulatorische, aber eben auch auf die hypertrophierende Funktion des NO, das enzymatisch aus dem Arginin gebildet wird, hingewiesen worden. Eine Häufung der Erkrankung ist in den gemäßigten Zonen beobachtet worden, jedoch sind die präpathologischen Faktoren nach wie vor unbekannt.

Die vorherrschende Therapie bei der MS ist die Zurückdrängung der Immunreaktion durch den Einsatz von Immunsuppresiva, wie z.B. Steroiden, Azathioprin und Interferonen beta-1b und beta-1a. Bezogen auf die postpubertäre MS ist ein völliger Stillstand der Krankheit nicht zu erreichen gewesen. Auch die bisherigen diätetischen Behandlungen konnten nicht zum Erfolg führen, da sie den verursachenden Metabolismus nicht berücksichtigten.

Nach hinreichender Klärung des Metabolismus durch die Erfinder sind gezielte Behandlungen möglich geworden (DE-B-4211878 und DE-U-29516585). Diese sind jedoch noch nicht in jedem Fall befriedigend.

Der Erfindung liegt die Aufgabe zugrunde, verbesserte Mittel auf Basis von Nahrungsmitteln oder Diätetika sowie Mittel zur parenteralen Behandlung zu entwickeln, durch die zugleich der Verbrauch und die auftretenden Nebenwirkungen der verwendeten Arzneimittel reduziert werden können.

Erfindungsgemäß bereitgestellt wird ein Mittel zur Behandlung von multipler Sklerose, das Proteine, Peptide und/oder Aminosäuren enthält, deren Gehalt an den Aminosäuren L-Arginin (Arg), L-Phenylalanin (Phe), L-Tyrosin (Tyr), Glycin (Gly), L-(+)-Glutaminsäure (Glu), L-Glutamin (Gln) und L-Asparagin (Asn) oder Gemischen davon für jede einzelne Aminosäure in einem definierten engen Bereich liegt mit einer Abweichung von ± 0,1 g der Aminosäuren Arg, Phe, Tyr, Gly und Glu und bei <0,01 g Gln und Asn jeweils pro 100 g Trockenmasse der Proteine, Peptide, Aminosäuren oder Gemische davon in dem Mittel, so daß die Gabe von Arg mit maximal 30 mg pro kg Körpermasse pro Tag, Phe mit maximal 70 mg pro kg Körpermasse pro Tag und Tyr mit maximal 40 mg pro kg Körpergewicht pro Tag, Gly mit maximal 70 mg pro kg Körpermasse pro Tag und Glu mit maximal 150 mg pro kg Körpermasse pro Tag gewährleistet ist.

Vorzugsweise liegt die Summe von Tyr und Phe bei maximal 100 mg pro kg Körpermasse pro Tag.

Gegenstand der Erfindung ist weiterhin ein Mittel zur Behandlung von multipler Sklerose, das Proteine, Peptide und/oder Aminosäuren enthält, deren Gehalt an den Aminosäuren Arg, Phe, Tyr, Gly, Glu, Gln und Asn oder Gemischen davon für jede einzelne Aminosäure in einem definierten engen Bereich liegt mit einer Abweichung von ± 0,1 g der Aminosäuren Arg, Phe, Tyr, Gly und Glu und bei <0,01 g Gln und Asn jeweils pro 100 g Trockenmasse der Proteine, Peptide, Aminosäuren oder Gemische davon in dem Mittel, hergestellt durch Behandlung mikrobiologischer, pflanzlicher oder tierischer Ausgangsstoffe, ausgewählt aus der Gruppe, die aus nichtpathogenen Hefen, Tiermilch, Tiermilchprodukten, Eiweiß, Leguminosen und Getreide besteht,
a) durch Behandlung eines Ausgangsstoffes über einen Zeitraum von 0,5 bis 10 Stunden im wäßrigen Medium unter Rühren bei einer Temperatur von 40 bis 90 °C bei einem pH-Wert im Bereich von 3 bis 9,
b) gegebenenfalls nachfolgende Behandlung mit einem reduzierenden Zucker, einem Enzym oder einem Acylierungs- oder Acetalisierungsmittel oder mit mehreren dieser Zusatzstoffe oder Biokatalysatoren nacheinander, und
c) nachfolgend Einstellung des pH-Wertes auf pH 4 - 6,
d) Trocknung des erhaltenen Produktes oder der abgetrennten festen und flüssigen Phase, und
e) gegebenenfalls Zusatz von Vitaminen, Mineralstoffen, Spurenelementen, Biostimulatoren oder Gemischen davon.

In einer bevorzugten Ausführungsform liegt beispielsweise ein Mittel vor, das aus einer nichtpathogenen Hefe durch Behandlung im wäßrigen Medium bei einem pH-Wert von 5-6 und bei etwa 30 bis 50 °C für einen für die Partialhydrolyse angemessenen Zeitraum von etwa 4 bis 6 Stunden und anschließende Trennung und Trocknung der festen und der flüssigen Phase oder beider hergestellt wird.

In einer weiteren Ausführungsform der Erfindung betrifft diese ein Mittel, dem nach der Behandlung im wäßrigen Medium über einen Zeitraum von 2 bis 4 Stunden bei 40 bis 90 °C ein reduzierendes Mono- oder Disaccharid wie Fructose, Glucose, Lactulose, Maltose oder Isomaltose zugesetzt wird, und die bei 50 bis 60 °C ablaufende Maillard-Reaktion bis zur Bildung von Prämelanoidinen geführt wird. Diese Ausführungsform der Erfindung führt zu einer teilweisen oder vollständigen Blockierung der Stickstoffunktion besonders in den freien Aminosäuren und damit zur Limitierung bestimmter Aminosäuren und kann für verschiedene Ausgangsstoffe besonders vorteilhaft sein.

In einer weiteren Ausführungsform der Erfindung betrifft diese ein Mittel, dem nach der Behandlung im wäßrigen Medium über einen Zeitraum von 2 bis 10 Stunden bei 20 bis 90 °C ein Enzym zugesetzt wird, ausgewählt unter Trypsin, Papain, Chymotrypsin und Clostripain, und die bei 25 bis 35 °C ablaufende enzymatische Reaktion bis zu einem Abbaugrad der Proteine im Bereich von 40 bis 60 % geführt wird, bezogen auf die durchschnittliche Molmasse des eingesetzten Ausgangsstoffes. Diese Enzyme haben Wirkungsoptima hinsichtlich ihrer pH-Werte, z.B. Trypsin bei pH 7,6, Chymotrypsin bei pH 7,8, Clostripain bei pH 7,5 und Papain bei pH 6,2 mit Toleranzen von etwa ±0,3. Die Zugabe dieser genannten Enzyme führt gewollt besonders zu Peptiden mit terminalem Arg, wobei eine Fraktionierung des entstandenen Peptid-Aminosäuregemisches durch Dialyse an geeigneten Dialysemembranen erfolgen kann.

In einer weiteren Ausführungsform der Erfindung betrifft diese ein Mittel, dem nach der Behandlung im wäßrigen Medium über einen Zeitraum von 2 bis 10 Stunden bei 20 bis 90 °C ein Acylierungs- oder Acetalisierungsmittel zugesetzt wird, ausgewählt unter Essigsäureanhydrid, Bersteinsäureanhydrid, Maleinsäureanhydrid, Fettsäureestern und Ketosäuren. Diese Ausführungsform der Erfindung führt ebenfalls zu einer teilweisen oder vollständigen Blockierung der Stickstoffunktionen in den freien Aminosäuren oder terminalen Aminosäuren in Peptiden und Proteinen und damit zur Limitierung bestimmter Aminosäuren.

Die in den letzten drei Ausführungsformen der Erfindung beschriebenen Verfahrensmaßnahmen, die zu einem erfindungsgemäßen Mittel mit verringertem Gehalt an in der Menge zu begrenzenden Aminosäuren führen, können einzeln durchgeführt oder miteinander kombiniert werden. So kann beispielsweise zuerst eine Behandlung mit einem Enzym und anschließend die Zugabe eines Zuckers erfolgen, oder es kann zuerst ein Enzym zugesetzt werden und anschließend acyliert werden.

Bei Einsatz von Hefen oder Zugabe von Enzymen ist nach der Beendigung der Reaktion eine kurzzeitige Erwärmung auf 70 bis 100 °C zweckmäßig bzw. erforderlich, um die Enzyme oder Mikroorganismen zu inaktivieren.

Als Ausgangsstoffe werden vorzugsweise Hefen eingesetzt, wie Bäckerhefe (Saccharomyces cerevisiae), Brauereihefe oder Weinhefe. Bevorzugte Tiermilch ist Stutenmilch oder Büffelmilch oder Ziegenmilch. Ein vorteilhaftes Tiermilchprodukt ist Labmolke, Sauermolke, Molkenprotein als Konzentrat, Joghurt oder Kefir. Als Eiweiß wird Eiweiß von Eiern, vorzugsweise Eiklarpulver eingesetzt.

Bevorzugte Leguminosen sind reife und gelagerte Ackerbohnen (Vicia faba), insbesondere weiß blühende Ackerbohnen. Bevorzugt sind auch reife Sojabohnen.

Bevorzugte Getreide sind Hirse und Amaranth.

Gegenüber dem Stand der Technik war es überraschend, daß ein Produkt bereitgestellt werden kann, dessen Konzentration an Arginin, Phenylalanin, Tyrosin, Glycin, Glutamin oder Asparagin oder Gemischen davon in einem ganz engen Schwankungsbereich liegt. Weiterhin war es überraschend, daß die genaue Einhaltung eines Temperatur/Zeit/pH-Wert-Regimes zu Produkten führt, die einen gewünschten Gehalt an Arg, Phe, Tyr, Gly, Glu, Gln oder Asn oder Gemischen davon in dem für die Verabreichung wichtigen Bereich von Arg = 10 - 30 mg, Phe = 10 - 70 mg und Tyr = 5 - 40 mg sowie Gly = 10 - 70 mg und Glu = 30 - 150 mg, jeweils pro kg Körpermasse eines Patienten pro Tag aufweisen, sowie praktisch Gln- und Asn-frei sind. Dadurch wird es möglich, das erfindungsgemäße Mittel zur Behandlung von multipler Sklerose gezielt auch in jedem Einzelfall einsetzen zu können. Eine besonders vorteilhafte Wirkung des erfindungsgemäßen Mittels besteht darin, daß bei der Nutzung das parenteral anzuwendende Mittel eine Anpassung an die chronobiologische Spezifik des einzelnen Patienten erlaubt und dadurch eine zusätzliche Wirkungssteigerung möglich ist.

Es ist außerdem möglich, in Intervallbehandlungen genaue Dosierungen vorzunehmen, wozu auch die zeitlich begrenzte erhöhte Gabe ausgewählter Vitamine bei kontrollierter Aminosäuremenge gehört.

Beispielsweise kann die Vitamin-B12-Gabe um das 3- bis 10-fache bis zu 1000 µg pro Tag für 1 bis 3 Tage erhöht werden, wenn das Mittel das 1,5- bis 2-fache, d.h. bis zu 250 mg Vitamin-B6 in der parenteralen Applikationsform für diesen Zeitraum enthält.

Zu beachten ist, daß Glu Neurotransmitter-Funktion besitzt und Ammoniak aus den Zellen auszuschleusen vermag, andererseits eine zu große Menge an Glu wegen der Energiebedürftigkeit des spezifischen Glutamat-Transportes durch die Blut-Hirn-Schranke und der potentiellen Neurotoxizität von überschüssigem Glutamin zu vermeiden ist.

Die Einstellung des Aminosäuregehaltes bei dem erfindungsgemäßen Mittel innerhalb der festgelegten Grenzen kann auch durch Vermischen verschiedener Fraktionen, Zwischenprodukte oder Endprodukte erfolgen, vorteilhaft besonders dann, wenn diese neben den Peptiden einen hohen Anteil an freien Aminosäuren enthalten.

Weiterhin kann das erfindungsgemäße Mittel Protonendonatoren wie Algin- oder Pektinsäure oder Carboxymethylcellulose bis zu einer Menge von 2 Gew-% enthalten, bezogen auf die Gesamtmenge der Proteintrockenmasse. Die Protonendonatoren katalysieren einerseits die Bildung von Ornithin und Harnstoff aus Arginin, und andererseits binden sie den im Enddarm infolge Urease-Wirkung entstandenen Ammoniak.

Das Mittel kann auch Galactosido-Schwefelsäuren wie Carrageenane und Agar-Agar bis zu einer Menge von 2 Gew-% enthalten, vorzugsweise 0,3 bis 0,8 Gew-%, bezogen auf die Gesamtmenge der Proteintrockenmasse.

Das erfindungsgemäße Mittel kann enteral oder parenteral eingesetzt werden. Bei der enteralen Applikation kann eine Kombination mit Ballaststoffen bis zu einer Menge von 2 Gew-%, bezogen auf die Gesamtmenge, erfolgen, wobei die Ballaststoffe ausgewählt sind unter Xanthan, Johannisbrotkern- oder Guarkernmehl, Gummi arabicum sowie modifizierten Stärken (z.B. Phosphatstärken), modifizierten Cellulosen, modifizierten Pektinen oder modifizierten Alginaten; auch ein Zusatz von L-Selenomethionin ist möglich.

Die parenterale Applikation als weitere Ausführungsform der Erfindung kann als Lösung oder stabile Suspension zusammen mit physiologischer Kochsalz- oder Pufferlösung erfolgen, wobei Ausgangspunkt ein Gemisch der oben genannten erfindungswesentlichen Aminosäuren in den genannten Grenzen, gegebenenfalls unter Zusatz von Vitaminen, insbesondere Vitamin-B12 und -B6, Spurenelementen, insbesondere Kupfer und Zink sowie Myoinositol als Biostimulator ist. Die Herstellung kann durch Vermischung der einzelnen Aminosäuren und Zusatzkomponenten erfolgen.

Mit den erfindungsgemäßen Mitteln wird neben den bereits genannten Vorteilen die Möglichkeit eröffnet, daß ein Patient selbst in die Lage versetzt wird, nach kurzer Einweisung eigenverantwortlich aus den erfindungsgemäßen Mittel eines oder mehrere auszuwählen und einzusetzen und damit nicht nur den Stillstand, sondern eine wesentliche Besserung der Erkrankung zu erreichen. Dabei ist es aus ernährungsphysiologischer Sicht empfehlenswert, die Mittel in Verbindung mit einer bilanzierten oder teilbilanzierten Diät anzuwenden, wie beispielsweise in Klein et al., Texte zum Lebensmittelrecht, Hamburg 1994, beschrieben.

Mit der Erfindung ist es insbesondere möglich, den intertestitinal gebildeten Ammoniak, das sind etwa 50 % der Gesamtmenge des NH₃, in unterschiedlichem Maße, aber möglichst vollständig zu binden, wobei die Wirkung gegebenenfalls durch zusätzliche Gaben von Lactulose oder Benzoat bzw. Phenylacetat verstärkt werden kann. Für die Ammoniak-Bindung kann auch die oben angeführte wahlweise Zugabe von Protonendonatoren, wie Algin- oder Pektinsäure oder Carboxymethylcellulose oder Galaktosido-Schwefelsäuren vorteilhaft sein.

Das erfindungsgemäße Mittel fungiert, um eine kontrollierte und dosierte Zuführung bestimmter Aminosäuren zu gewährleisten, über einen bestimmten Zeitraum als alleiniges Nahrungsmittel, gegebenenfalls einer parenteralen Versorgungsform nachfolgend. Das erfindungsgemäße Mittel, bestehend aus Mischungen ausgewählter Aminosäuren, Kohlenhydrate, Vitamine, Biostimulatoren, Mineralstoffen und Spurenelemente, übernimmt in der parenteralen Applikationsform die Versorgung des geschädigten Organismus anstelle von Eiweiß, Peptiden, oder Komponenten davon. In einer Ausführungsform wird zur kurzzeitigen Entlastung des geschädigten Stoffwechsels ein Mittel bereitgestellt, das dadurch gekennzeichnet ist, daß neben den Aminosäuren Arg, Asn, Gln und Gly auch der Gehalt an Phe und Tyr auf Null reduziert wird, und zwar bei deutlcher Erhöhung des Gehaltes an Vitamin-B6 von 100 auf 250 mg und Vitamin-B12 von 80 µg auf 1000 µg, wobei der Gehalt an den biostimulierenden Ionen Zink und Kupfer 27 mg bzw. 6 mg +/- 5% beträgt, allgemein bezogen auf 100 g Trockenmasse des Mittels. Das Mittel für die kontrollierte enterale Zufuhr, bestehend aus ausgewählten Aminosäuren und ausgewählten Zusätzen, darunter auch L-Selenomethionin, dient der Einstellung des Organismus auf eine Versorgung mit dem Mittel, das in der Hauptsache neben den Zusatzstoffen vor allem aus Proteinen und Peptiden besteht.

Das Mittel kann aber auch, den Zeitraum der Schubsymptomatik ausgenommen, neben einer ausgewogenen Diät entsprechend den ärztlichen Vorschriften und entsprechend den Nahrungsgewohnheiten des MS-Patienten von diesem aufzunehmen sein. Darüber hinaus kann das Mittel auch in anderen Nahrungsmitteln vermischt vorliegen und gegebenenfalls bei Bedingungen verarbeitet und dann in dieser Form verabreicht werden, wenn dabei die Gesamtmenge an zu limitierenden Komponenten nicht überschritten wird.

Die Erfindung soll nachstehend durch Beispiele näher erläutert werden.

### Beispiel 1

1 kg einer Hefe von Saccharomyces-Stämmen wurde in 300 ml Wasser mit einer Temperatur von etwa 50 °C, das etwa 15 % Ethanol enthielt, aufgenommen. Der pH-Wert wurde mit einer Säure, vorzugsweise einer mehrbasigen Säure, hier Phosphorsäure in Verbindung mit Citronensäure, auf 5,5 bis 6 eingestellt und die Suspension bei einer Temperatur von etwa 50 °C über eine Zeitraum von etwa 3 Stunden gerührt.

Danach wurde die Maische auf etwa 75°C erwärmt und der feste Rückstand abgetrennt. Sowohl die flüssige Phase als auch der feste Rückstand können getrocknet werden und einzeln oder gemeinsam durch weitere Zusätze, wie z.B. mit jeweils 80 µg Vitamin-B12 in Verbindung mit 100 mg Vitamin B-6, versetzt werden.

Das Produkt aus der flüssigen Phase enthielt u. a. die Aminosäuren Arg, Phe, Tyr, Glu, Gln und Asn in Mengen, wie in Tabelle 1 unter (1) angegeben.

**Tabelle 1**

| Aminosäuregehalte in der flüssigen Phase (1) und im Rückstand (2) in Gramm, jeweils bezogen auf 100 g Trockenmasse (Schwankungsbreite ±0,1 g) | | |
|---|---|---|
| Aminosäure | (1) | (2) |
| Arg | 1,5 | 1,0 |
| Phe | 3,0 | 2,0 |
| Tyr | 0,8 | 0,5 |
| Gly | 2,9 | 2,0 |
| Glu | 7,8 | 7,0 |
| Gln | 0 | 0 |
| Asn | 0 | 0 |

### Beispiel 2

Der feste Rückstand von Beispiel 1 wurde in auf 30 bis 50 °C erwärmtes Wasser suspendiert. Danach ließ man die Suspension für etwa 6 Stunden bei dieser Temperatur stehen. Der pH-Wert lag bei pH 6-7. Schließlich wurde kurzzeitig auf 70 bis 100 °C erwärmt und filtriert. Die Aufarbeitung und der Vitaminzusatz erfolgte wie im Beispiel 1. Das Produkt enthielt die Aminosäuren der Tabelle 1 in den in Spalte (2) angegebenen Mengen.

### Beispiel 3

1 kg einer Hefe von Saccharomyces-Stämmen wurde in 300 ml Wasser mit einer Temperatur von etwa 40 °C, das bis zu 15 % Ethanol enthalten kann, gewaschen. Danach wurde der Rückstand in Wasser bei etwa 40 bis 45 °C aufgenommen und der pH-Wert auf 5,5 bis 6,0 eingestellt und die Suspension bei dieser Temperatur über einen Zeitraum von etwa 6 Stunden gerührt. Nach kurzer Erwärmung auf etwa 75°C wurde nach 60 Minuten abdekantiert und der abgetrennte überstand unter Rühren portionsweise mit 200 g frisch gefälltem Hydroxylapatit behandelt. Anschließend wurde filtriert und durch Dialyse gegen destilliertes Wasser aufkonzentriert. Das Produkt enthielt die in Tabelle 2 aufgeführten Aminosäuren in den dort angegebenen Mengen.

**Tabelle 2**

| Aminosäuregehalte einer über Hydroxylapatit aufgearbeiteten Hefe von Saccharomyces-Stämmen in g, jeweils bezogen auf 100 g Trockenmasse (Schwankungsbreite ±0,1 g) | |
|---|---|
| Aminosäure | Gehalt |
| Arg | 1,5 |
| Phe | 2,5 |
| Tyr | 0,7 |
| Gly | 2,5 |
| Glu | 6,0 |
| Gln | 0 |
| Asn | 0 |

### Beispiel 4

Der filtrierte überstand aus Beispiel 1 wurde unter Rühren mit folgenden Biopolymeren, Süßungsmitteln, Spurenelementen, Vitaminen und mit Selenomethionin versetzt (berechnet auf 100 g Trockenmasse)

| | |
|---|---|
| Alginat | 1 g |
| Dextran | 0,5 g |
| Glucose/Xylitol¹ | 6,2 g |
| Vitamin B6 | 100 mg |
| Vitamin B12 | 80 µg |
| Kupfer(II)acetat² | 11 mg |
| Zinkacetat² | 50 mg |
| L-Selenomethionin | 50 µg |

| | |
|---|---|
| (¹ anzuwenden bei Diabetes; | |
| ² Stimulatoren für die Wirksamkeit von Vitamin B6 uns B12) | |

und schließlich unter milden Bedingungen gefriergetrocknet.

### Beispiel 5

Zur noch flüssigen Mischung aus Beispiel 4 fügte man 1 % Carboxymethylcellulose, bezogen auf die Gesamtmasse dieser Mischung. Die verdickte Mischung wurde unter milden Bedingungen gefriergetrocknet.

### Beispiel 6

Zur noch flüssigen Mischung aus Beispiel 4 fügte man 0,5 % Carrageenan hinzu, bezogen auf die Gesamtmasse dieser Mischung. Die gelierende Mischung wurde unter milden Bedingungen gefriergetrocknet.

### Beispiel 7

1 kg Molkenpulver wurde für 8 Stunden in 0,1 %iger Natronlauge aufgenommen, dabei wurde der pH-Wert des Systems auf pH 8 eingestellt. Man titrierte mit verdünnter Salzsäure bis zum Neutralpunkt und versetzte die entstandene Suspension mit 4 g Glucose. Die so gestartete Maillard-Reaktion wurde nach 6 Stunden durch eine abschließende Ultrafiltration weitgehend unterbrochen. Das aufkonzentrierte Produkt wurde mit 80 µg Vitamin-B12 und 100 mg Vitamin-B6 versetzt und danach gefriergetrocknet.

### Beispiel 8

Es wurde wie im Beispiel 7 gearbeitet, jedoch anstelle von Glucose wurden 2,5 g Fructose eingesetzt. Das Produkt kann dann auch von MS-Patienten angewendet werden, die zugleich an Diabetes erkrankt sind.

### Beispiel 9

Man versetzte 1 kg Stutenmilchpulver mit 0,1 %iger Natronlauge bis zum pH-Wert von 7,5 - 8 und nach 8 Stunden wurde auf 70 - 80 °C erwärmt. Nach der pH-Wert-Einstellung auf 6,2-6,5 hydrolysierte man das Protein/Peptid-Gemisch in Gegenwart von Papain bis zu einem durchschnittlichen Peptidmolekulargewicht von 2000 Dalton. Danach wurde das Hydrolysat mit Acetanhydrid in Gegenwart von 1N Natronlauge so acyliert, daß der pH-Wert in den Grenzen von 5,5 - 7,5 gehalten wurde. Das acetylierte Produkt trennte man nach Einengen der Suspension als Präzipitat ab.

### Beispiel 10

1 kg Eiklarpulver wurden für 8 Stunden in einer solchen Menge 0,1 %iger Natronlauge suspendiert, daß sich ein pH-Wert von 8 einstellte. Danach versetzte man mit der berechneten Menge Trypsin und führte die Hydrolyse bis zu einem Abbaugrad von 40 - 60 % des eingesetzten Proteinrohstoffes. Danach wurde filtriert und der Arginin-Gehalt im Hydrolysat ermittelt und mittels der berechneten notwendigen Arginase-Aktivität das gewünschte Protein-Präparat mit einem Argininwert <0,1 % erhalten, welches zweckmäßigerweise bis auf einen Wassergehalt unter 10 % getrocknet wurde.

### Beispiel 11

1 Kg Eiklarpulver wurden für 8 Stunden in einer solchen Menge 0,1 %iger Natronlauge suspendiert, daß sich ein pH-Wert von 8 einstellte. Danach versetzte man mit der berechneten Menge Trypsin und führte die Hydrolyse bis zu einem Abbaugrad von 40 - 60 % des eingesetzten Proteinrohstoffes. Danach wurden 5 g Glucose hinzugegeben. Nach der pH-Wert-Einstellung auf etwa 5,5 erfolgte nach 4 Stunden die Abtrennung und Trocknung des Produktes, dem schließlich 80 µg Vitamin-B12 und 100 mg Vitamin-B6 hinzugefügt wurden.

### Beispiel 12

1 kg Eiklarpulver wurden für 8 Stunden in einer solchen Menge 0,1 %iger Natronlauge suspendiert, daß sich ein pH-Wert von 8 einstellte. Danach versetzte man mit der berechneten Menge Trypsin und führte die Hydrolyse bis zu einem Abbaugrad von 40 - 60 % des eingesetzten Proteinrohstoffes. Danach wurden 2,5 g Fructose hinzugefügt. Nach der pH-Wert-Einstellung auf etwa 5,5 erfolgte nach 4 Stunden die Abtrennung und Trocknung des Produktes, dem schließlich 80 µg Vitamin-B12 und 100 mg Vitamin-B6 hinzugefügt wurden. Das Produkt kann auch von MS-Patienten angewendet werden, die zugleich an Diabetes erkrankt sind.

### Beispiel 13

1 kg von wenigstens über ein halbes Jahr gelagerten und gut getrockneten weißen Bohnen wurden gemahlen und danach bei 110 - 130 °C hydrothermisch behandelt. Danach nahm man für eine Stunde in Wasser bei einem pH-Wert von 4,5 auf und filtrierte. Der Rückstand wurde in Wasser unter Beibehaltung des pH-Wertes mit 7 g Glucose bei 80 °C zur Reaktion gebracht und erneut von der Mutterlauge abgetrennt.

### Beispiel 14

1 kg der wenigstens über ein halbes Jahr gelagerten und gut getrockneten weißen Bohnen wurden gemahlen und danach bei 110 - 130 °C hydrothermisch behandelt. Danach nahm man für eine Stunde in Wasser bei einem pH-Wert von 4,5 auf und filtrierte. Der Rückstand wurde in Wasser unter Beibehaltung des pH-Wertes mit 4 g Fructose bei 80 °C zur Reaktion gebracht und erneut von der Mutterlauge abgetrennt. Das Produkt kann dann auch von MS-Patienten angewendet werden, die zugleich an Diabetes erkrankt sind.

### Beispiel 15

Für die parenterale Anwendung wurden 100 g eines Produktes mit der Aminosäure-Zusammensetzung und den Zusatzstoffen (Supplement) gemäß Tabelle 3 in 1000 ml einer physiologischen Kochsalzlösung eingetragen und in üblicher Weise sterilisiert und konfektioniert. Variante A ist für die normale Anwendung, Variante B für ein Entlastungsintervall.

**Tabelle 3**

| Aminosäure/Supplement | Variante A in g | Variante B in g |
|---|---|---|
| L-Alanin | 2,0 | 2,0 |
| L-Serein | 5,7 | 6,0 |
| L-Threonin | 8,0 | 7,0 |
| L-Valin | 11,0 | 8,5 |
| L-Leucin | 13,8 | 14,5 |
| L-Isoleucin | 11,3 | 12,0 |
| L-Asparaginsäure | 3,8 | 6,5 |
| L-(+)-Glutaminsäure | 7,5 | 6,5 |
| L-Lysin | 7,5 | 7,5 |
| L-Cystin | 0,5 | 2,0 |
| L-Methionin | 1,1 | 3,5 |
| L-Phenylalanin | 0,2 | - |
| L-Tyrosin | 0,1 | - |
| L-Prolin | 8,0 | 7,0 |
| L-Tryptophan | 3,0 | 3,5 |
| L-Histidin | 6,5 | 3,5 |
| Vitamin-B6 | 0,100 | 0,250 |
| Vitamin-B12 | 80µg | 1000µg |
| Myoinositol | 0,300 | 0,300 |
| Kupferacetat | 0,011 | 0,011 |
| Zinkacetat | 0,050 | 0,050 |

sowie weitere Vitamine und Salze

## Patentansprüche

1. Mittel zur metabolischen Behandlung von multipler Sklerose, **dadurch gekennzeichnet, daß** es Proteine, Peptide und Aminosäuren enthält, deren Gehalt an den Aminosäuren L-Arginin (Arg), L-Phenylalanin (Phe), L-Tyrosin (Tyr), Glycin (Gly), L-Glutaminsäure (Glu), L-Glutamin (Gln) und L-Asparagin (Asn) oder Gemischen davon für jede einzelne Aminosäure in einem definierten engen Bereich liegt, nämlich Arg=10-30 mg, Phe=10-70 mg, Tyr=5-40 mg, Gly =10-70 mg und Glu=3-150 mg, jeweils pro kg Körpermasse eines Patienten pro Tag und praktisch Gln- und Asn-frei, mit einer Abweichung von ± 0,1 g der Aminosäuren Arg, Phe, Tyr, Gly und Glu und bei <0,01 g Gln und Asn jeweils pro 100 g Trockenmasse der Proteine, Peptide, Aminosäuren oder Gemische davon in dem Mittel, und gegebenenfalls Vitaminen, Mineralstoffen, Spurenelementen oder Gemischen davon, so daß die Gabe von Arg mit maximal 30 mg, Phe mit maximal 70 mg und Tyr mit maximal 40 mg, Gly mit maximal 70 mg und Glu mit maximal 150 mg jeweils pro kg Körpermasse pro Tag gewährleistet ist.

2. Mittel zur metabolischen Behandlung von multipler Sklerose, **dadurch gekennzeichnet, daß** es Proteine, Peptide und Aminosäuren enthält, deren Gehalt an den Aminosäuren Arg, Phe, Tyr, Glu, Gly, Gln und Asn oder Gemischen davon für jede einzelne Aminosäure in einem definierten engen Bereich liegt, nämlich Arg=10-30 mg, Phe=10-70 mg, Tyr=5-40 mg, Gly=10-70 mg und Glu=3-150 mg, jeweils pro kg Körpermasse eines Patienten pro Tag und praktisch Gln- und Asn-frei, mit einer Abweichung von ± 0,1 g der Aminosäuren Arg, Phe, Tyr, Gly und Glu und bei <0,01 g Gln und Asn jeweils pro 100 g Trockenmasse der Proteine, Peptide, Aminosäuren oder Gemische davon in dem Mittel,
hergestellt durch Behandlung mikrobiologischer, pflanzlicher oder tierischer Ausgangsstoffe, ausgewählt aus der Gruppe, die aus nichtpathogenen Hefen, Tiermilch, Tiermilchprodukten, Eiweiß, Leguminosen und Getreide besteht,
a) durch Behandlung des Ausgangsstoffes über einen Zeitraum von 2 bis 10 Stunden im wäßrigen Medium unter Rühren bei einer Temperatur von 40 bis 90 °C bei einem pH-Wert im Bereich von 3 bis 9;
b) gegebenenfalls nachfolgende Behandlung mit einem reduzierenden Zucker, einem Enzym oder einem Acylierungs- oder Acetalisierungsmittel oder mit mehreren dieser Zusatzstoffe oder Biokatalysatoren nacheinander;
c) Einstellung des pH-Wertes auf pH 4 - 6;
d) Trocknung des Stoffes oder der abgetrennten festen und flüssigen Phase; und gegebenenfalls
e) Zusatz von Vitaminen, Mineralstoffen, Spurenelementen oder Gemischen davon.

3. Mittel nach Anspruch 1 oder 2, **dadurch gekennzeichnet, daß** die Hefe Bäckerhefe, Weinhefe oder Brauereihefe ist.

4. Mittel nach Anspruch 2, **dadurch gekennzeichnet, daß** der dem im wäßrigen Medium behandelten Produkt zugesetzte Zucker ausgewählt ist unter Fructose, Glucose, Lactulose, Maltose, Isomaltose und Gemischen davon.

5. Mittel nach einem der Ansprüche 2 bis 4, **dadurch gekennzeichnet, daß** dem im wäßrigen Medium behandelten Produkt ein Enzym zugesetzt wird, ausgewählt unter Trypsin, Papain, Chymotrypsin und Clostripain, und die Hydrolyse bis zu einem Abbaugrad des Proteins im Bereich von 40 bis 60 %, bezogen auf die mittlere Molmasse des eingesetzten Ausgangsstoffes, geführt wird.

6. Mittel nach einem der Ansprüche 2 bis 5, **dadurch gekennzeichnet, daß** dem im wäßrigen Medium behandelten Ausgangsstoff oder Produkt ein Polyphosphat zugesetzt wird.

7. Mittel nach einem der Ansprüche 1 bis 6, **dadurch gekennzeichnet, daß** es Ballaststoffe bis zu einer Menge von 2 Gew-% enthält, bezogen auf die Gesamtmenge, wobei die Ballaststoffe ausgewählt sind unter Xanthan, Johannisbrotkern- oder Guarkernmehl, Gummi arabicum sowie modifizierten Stärken, modifizierten Cellulosen, modifizierten Pektinen oder modifizierten Alginaten.

8. Mittel nach einem der Ansprüche 1 bis 6, **dadurch gekennzeichnet, daß** es Protonendonatoren, wie Carboxymethylcellulose, bis zu einer Menge von 2 Gew-% enthält, bezogen auf die Gesamtmenge.

9. Mittel nach einem der Ansprüche 1 bis 8, **dadurch gekennzeichnet, daß** es Galactosido-Schwefelsäuren, wie Carrageenane oder Agar-Agar, bis zu einer Menge von 2 Gew-% enthält, vorzugsweise 0,3 bis 0,8 Gew-%, bezogen auf die Gesamtmenge.

10. Mittel nach einem der Ansprüche 1 bis 9, **dadurch gekennzeichnet, daß** es Selenomethionin enthält.

11. Mittel nach Anspruch 1, **dadurch gekennzeichnet, daß** es in einer parenteral verabreichbaren Form vorliegt, gegebenenfalls zusammen mit Vitamin-B12, Vitamin-B6, den Spurenelementen Kupfer und Zink und Diostimulatoren sowie Zucker, ausgewählt unter Fructose, Glucose, Lactulose, Maltose, Isomaltose und Gemischen davon.

12. Mittel nach einem der Ansprüche 1 bis 11, **dadurch gekennzeichnet, daß** der Gehalt an Vitamin-B12 um das 3- bis 12-fache erhöht ist und der Gehalt an Vitamin-B6 um das 1,5- bis 2,5-fache erhöht ist.

13. Nahrungsmittel, enthaltend das Mittel nach einem der Ansprüche 1 bis 12.

## Claims

1. An agent for metabolic treatment of multiple sclerosis, comprising proteins, peptides and amino acids, and the amount of the amino acids L-arginine (Arg), L-phenylalanine (Phe), L-tyrosine (Tyr), glycine (Gly), L-glutamic acid (Glu), L-glutamine (Gln) and L-asparagine (Asn) or mixtures thereof is within a narrowly defined range for each individual amino acid, namely Arg = 10-30 mg, Phe = 10-70 mg, Tyr = 5-40 mg, Gly = 10-70 mg and Glu = 3-150 mg, each per kg body weight of a patient per day and being practically free of Gln and Asn, with a range of variation of ±0.1 g of the amino acids Arg, Phe, Tyr, Gly and Glu and <0.01 g Gln and Asn, each per 100 g dry solids of the proteins, peptides, amino acids or mixtures thereof in the agent, plus optional vitamins, minerals, trace elements or mixtures thereof, so as to guarantee administration of Arg in a maximum dose of 30 mg per kg body weight per day, Phe in a maximum dose of 70 mg per kg body weight per day, Tyr in a maximum dose of 40 mg per kg body weight per day, Gly in a maximum dose of 70 mg per kg of body weight per day, and Glu in a maximum dose of 150 mg per kg body weight per day.

2. An agent for metabolic treatment of multiple sclerosis, comprising proteins, peptides and amino acids, with the content of amino acids Arg, Phe, Tyr, Gly, Glu, Gln and Asn or mixtures thereof for each individual amino acid being within a narrowly defined range, namely Arg = 10-30 mg, Phe = 10-70 mg, Tyr = 5-40 mg, Gly = 10-70 mg and Glu = 3-150 mg, each per kg body weight of a patient per day and being practically free of Gln and Asn, with a range of variation of ± 0.1 g, of the amino acids Arg, Phe, Tyr, Gly and Glu and <0.01 g Gln and Asn, each per 100 g dry solids of the proteins, peptides, amino acids or mixtures thereof in the agent, prepared by treating microbiological, plant or animal starting materials selected from the group of nonpathogenic yeasts, animal milk, animal milk products, proteins, legumes and grains,
a) by treating the starting material in an aqueous medium while stirring at a temperature of 40 to 90 °c at a pH in the range of 3-9 for a period of two to ten hours;
b) optionally performing a subsequent treatment with areducing sugar, an enzyme or an acylating or acetalizing agent or with several such additives or biocatalysts in succession;
c) adjusting the pH to 4-6;
d) drying the substance or the separated solid and liquid phases; and
e) optionally adding vitamins, minerals, trace elements or mixtures thereof.

3. An agent according to Claim 1 or 2, wherein the yeast is baker's yeast, wine yeast or brewer's yeast.

4. An agent according to Claim 2, wherein the sugar added to the product treated in the aqueous medium is selected from the group consisting of fructose, glucose, lactulose, maltose, isomaltose and mixtures thereof.

5. An agent according to one of Claims 2 to 4, wherein the enzyme is added to the product treated in the aqueous medium and the enzyme is selected from the group consisting of trypsin, papain, chymotrypsin and clostripain, and hydrolysis is performed to a degree of degradation of the protein in the range of 40 % to 60 %, based on the average molecular weight of the starting material.

6. An agent according to one of Claims 2 to 5, wherein a polyphosphate is added to the starting material or product treated in the aqueous medium.

7. An agent according to one of Claims 1 to 6, wherein the agent comprises dietary fiber in an amount of up to 2 wt %, based on the total amount, said dietary fiber being selected from the group consisting of xanthan, carob flour, guar flour, gum arabic, modified starches, modified celluloses, modified pectins and modified alginates.

8. An agent according to one of Claims 1 to 6, wherein the agent comprises proton donors such as carboxymethylcellulose in an amount of up to 2 wt %, based on the total amount.

9. An agent according to one of Claims 1 to 8, wherein the agent comprises galactosidosulfuric acids such as carrageenans or agar in an amount of up to 2 wt %, preferably of 0.3 to 0.8 wt %, based on the total quantity.

10. An agent according to one of Claims 1 to 9, wherein the agent comprises selenomethionine.

11. An agent according to Claim 1, wherein it is in a form suitable for parenteral administration, optionally together with vitamin B12, vitamin B6, the trace elements copper and zinc and biostimulators, as well as sugars selected from the group consisting of fructose, glucose, lactulose, maltose, isomaltose and mixtures thereof.

12. An agent according to one of Claims 1 to 11, wherein the vitamin B12 content is increased by a factor of 3 to 12 and the vitamin B6 content is increased by a factor of 1.5 to 2.5.

13. A nutritional agent comprising an agent according to one of Claims 1 to 12.

## Revendications

1. Agent pour le traitement métabolique de la sclérose en plaques **caractérisé en ce qu'**il contient des protéines, des peptides et des acides aminés, dont la teneur en acides aminés L-arginine (Arg), L-phénylalanine (Phe), L-tyrosine (Tyr), Glycine (Gly), acide L-glutamique (Glu), L-glutamine (Gln) et L-asparagine (Asn) ou en mélanges de ces acides aminés se trouve pour chaque acide aminé comprise dans un domaine étroit défini, à savoir Arg=10-30 mg, Phe=10-70 mg, Tyr=5-40 mg, Gly=10-70 mg et Glu=3-150 mg par kg de masse corporelle pour un patient par jour et dont la teneur en Gln et Asn est pratiquement nulle, avec une variation de ± 0,1 g pour les acides aminés Arg, Phe, Tyr, Gly et Glu et inférieure à 0,01 g pour les acides aminés Gln et Asn pour 100 g de masse sèche de protéines, de peptides, d'acides aminés ou de leur mélange dans l'agent et **caractérisé en ce qu'**il contient le cas échéant des vitamines, des minéraux, des oligo-éléments ou des mélanges de ces substances, de telle sorte que l'administration d'une dose maximale d'Arg de 30 mg, de Phe de 70 mg et de Tyr de 40 mg, de Gly de 70 mg et de Glu de 150 mg par kg de masse corporelle et par jour soit assurée.

2. Agent pour le traitement métabolique de la sclérose en plaques, **caractérisé en ce qu'**il contient des protéines, des peptides et des acides aminés, dont la teneur en acides aminés Arg, Phe, Tyr, Glu, Gly, Gln et Asn ou en mélanges de ces acides aminés se trouve pour chaque acide aminé comprise dans un domaine étroit défini, à savoir Arg=10-30 mg, Phe=10-70 mg, Tyr=5-40 mg, Gly=10-70 mg et Glu=3-150 mg par kg de masse corporelle pour un patient par jour et dont la teneur en Gln et Asn est pratiquement nulle, avec une variation de ± 0,1 g pour les acides aminés Arg, Phe, Tyr, Gly et Glu et inférieure à 0,01 g pour les acides aminés Gln et Asn pour 100 g de masse sèche de protéines, de peptides, d'acides aminés ou de leur mélange dans l'agent, fabriqué par traitement de matières premières microbiologiques, végétales ou animales choisies parmi le groupe constitué des levures non pathogènes, du lait animal, des produits laitiers animaux, du blanc d'oeuf, des légumineuses et des céréales,
a) par traitement de la matière première pendant 2 à 10 heures dans un milieu aqueux par mélange à une température comprise entre 40 et 90 ° C à un pH compris entre 3 et 9 ;
b) le cas échéant traitement subséquent avec un sucre réducteur, une enzyme ou un agent d'acylation ou d'acétalisation ou avec plusieurs de ces additifs ou biocatalysateurs les uns après les autres ;
c) établissement d'un pH compris entre 4 et 6 ;
d) séchage de la matière ou des phases solides et liquides séparées et le cas échéant
e) ajout de vitamines, minéraux, oligo-éléments ou de mélanges de ces substances.

3. Agent selon les revendications 1 ou 2, **caractérisé en ce que** la levure est de la levure de boulanger, de la levure de vin ou de la levure de bière.

4. Agent selon la revendication 2, **caractérisé en ce que** le sucre ajouté au produit traité dans le milieu aqueux est choisi parmi le fructose, le glucose, le lactulose, le maltose, l'isomaltose et des mélanges de ces sucres.

5. Agent selon l'une quelconque des revendications 2 à 4, **caractérisé en ce qu'**une enzyme est ajoutée au produit traité dans le milieu aqueux, choisie parmi la trypsine, la papaïne, la chymotrypsine et la clostripaïne et **en ce que** l'hydrolyse est pratiquée jusqu'à un degré de dégradation de la protéine de 40 à 60 % de la masse molaire moyenne de la matière première mise en oeuvre.

6. Agent selon l'une quelconque des revendications 2 à 5, **caractérisé en ce qu'**un polyphosphate est ajouté à la matière première ou au produit traité dans le milieu aqueux.

7. Agent selon l'une quelconque des revendications 1 à 6, **caractérisé en ce qu'**il contient jusqu'à 2 % en poids de substances de lest rapportées à la quantité totale, les substances de lest étant choisies parmi le xanthane, la farine de graines de caroube ou la farine de graines de guar, la gomme arabique ainsi que parmi des amidons modifiés, des celluloses modifiées, des pectines modifiées ou des alginates modifiées.

8. Agent selon l'une quelconque des revendications 1 à 6, **caractérisé en ce qu'**il contient jusqu'à 2 % en poids de donneurs de protons comme la carboxyméthylcellulose, rapportés sur la quantité totale.

9. Agent selon l'une quelconque des revendications 1 à 8, **caractérisé en ce qu'**il contient jusqu'à 2 % en poids d'acides galactosido-sulfuriques comme la carragheenine ou l'agar-agar, de préférence 0,3 à 0,8 % en poids rapportés sur la quantité totale.

10. Agent selon l'une quelconque des revendications 1 à 9, **caractérisé en ce qu'**il contient de la sélénométhionine.

11. Agent selon la revendication 1, **caractérisé en ce qu'**il se présente sous une forme administrable par voie parentérale, le cas échéant avec de la vitamine B12, de la vitamine B6, les oligo-éléments cuivre et zinc et des biostimulateurs ainsi que du sucre, choisi parmi le fructose, le glucose, le lactulose, le maltose, l'isomaltose et des mélanges de ces sucres.

12. Agent selon l'une quelconque des revendications 1 à 11, **caractérisé en ce que** la teneur en vitamine B12 est 3 à 12 fois supérieure et **en ce que** la teneur en vitamine B6 est 1,5 à 2,5 fois supérieure.

13. Produit alimentaire contenant l'agent selon l'une quelconque des revendications 1 à 12.
